# EUROPÄISCHE PATENTSCHRIFT

(11) **EP 3 193 309 B1**
(45) Veröffentlichungstag und Bekanntmachung des Hinweises auf die Patenterteilung: **16.08.2023**
(21) Anmeldenummer: 16200397.4
(22) Anmeldetag: 24.11.2016
(51) Int. Cl.: G06T 15/04, G06T 17/20, G06T 19/00, A61B 6/03, A61B 6/00

(54) **PERSPEKTIVISCHES DARSTELLEN DER SCANBEREICHSGRENZE EINER MEDIZINISCHEN BILDGEBUNGSVORRICHTUNG**
PERSPECTIVE-BASED REPRESENTATION OF THE SCAN AREA BOUNDARY OF A MEDICAL IMAGING DEVICE
REPRÉSENTATION EN PERSPECTIVE DE LA LIMITE DE LA ZONE DE BALAYAGE D'UN DISPOSITIF D'IMAGERIE MÉDICALE

(30) Priorität: 12.01.2016 DE 102016200225
(43) Veröffentlichungstag der Anmeldung: 19.07.2017
(73) Patentinhaber: Siemens Healthcare GmbH, 91052 Erlangen (DE)
(72) Erfinder: Wels, Michael, 96047 Bamberg (DE); Distler, Ferdinand, 91056 Erlangen (DE); Hannemann, Thilo, 91052 Erlangen (DE); Sühling, Michael, 91052 Erlangen (DE); Wimmer, Andreas, 91301 Forchheim (DE)

(56) Entgegenhaltungen:
- DE-A1-102012 201 798
- US-A1- 2015 213 646
- SINGH VIVEK ET AL: "Estimating a Patient Surface Model for Optimizing the Medical Scanning Workflow", NETWORK AND PARALLEL COMPUTING; [LECTURE NOTES IN COMPUTER SCIENCE; LECT.NOTES COMPUTER], SPRINGER INTERNATIONAL PUBLISHING, CHAM, Bd. 8673 Chap.59, Nr. 558, 14. September 2014 (2014-09-14), Seiten 472-479, XP047312311, ISSN: 0302-9743 ISBN: 978-3-642-38492-9
- SUMA EVAN A ET AL: "Rapid generation of personalized avatars", 2013 IEEE VIRTUAL REALITY (VR), IEEE, 18. März 2013 (2013-03-18), Seite 185, XP032479413, ISSN: 1087-8270, DOI: 10.1109/VR.2013.6549424 ISBN: 978-1-4673-4795-2 [gefunden am 2013-06-27]
- Vivek Rajan, Satheesh Subramanian, Damin Keenan et al: "A Realistic Video Avatar System for Networked Virtual Environments", Electronic Visualization LaboratoryUniversity of Illinois at Chicago, Chicago, IL, USA , 2002, XP002770331, Gefunden im Internet: URL:http://citeseerx.ist.psu.edu/viewdoc/s ummary?doi=10.1.1.18.6987&rank=1http://cit eseerx.ist.psu.edu/viewdoc/download?doi=10 .1.1.18.6987&rep=rep1&type=pdf [gefunden am 2017-05-18]
- WILLIAMS N ET AL: "Automatic image alignment for 3D environment modeling", COMPUTER GRAPHICS AND IMAGE PROCESSING, 2004. PROCEEDINGS. 17TH BRAZIL IAN SYMPOSIUM ON CURITIBA, PR, BRAZIL 17-20 OCT. 2004, PISCATAWAY, NJ, USA,IEEE, 17. Oktober 2004 (2004-10-17), Seiten 388-395, XP010737848, DOI: 10.1109/SIBGRA.2004.1352985 ISBN: 978-0-7695-2227-2

## Beschreibung

Die Erfindung betrifft ein Verfahren und eine Vorrichtung zum perspektivischen Darstellen eines virtuellen, innerhalb einer Realszene angeordneten Szenebestandteils, insbesondere einer Scanbereichsgrenze, unter Zuhilfenahme von der Realszene entsprechender Tiefeninformation.

Vor einer Messung mit einer medizinischen Bildgebungsanlage ist es in der Regel erforderlich, zunächst einen sogenannten "Scanbereich" festzulegen, d.h. den Bereich, von dem während eines "Scanvorgangs" Bilddaten aufgenommen bzw. Rohdaten akquiriert werden sollen, um daraus die gewünschten Bilddaten zu erzeugen.

Oft erfolgt die Festlegung des Scanbereichs mit Hilfe eines Topogramms, welches einer klassischen Röntgenprojektion aus nur einer Projektionsrichtung entspricht und welches die für die "diagnostische" Bildgebung interessierende Region (z.B. Herz und Lunge eines Untersuchungsobjektes), abbildet. Dem Topogramm können dann Start- und Endpunkt für die eigentliche Bildakquisition z.B. manuell überlagert werden. Der Start- und Endpunkt des Scanbereichs für eine Bilddatenerfassung eines dreidimensionalen Volumens entspricht einer Ebene im Raum, die in aller Regel senkrecht zur Längsachse des Untersuchungsobjektes repräsentiert wird. Die Aufnahme eines Topogramms verursacht jedenfalls eine zusätzliche Dosisbelastung, was per se dem in der Radiologie allgemein vorherrschenden ALARA-Prinzip entgegensteht.

Im Folgenden wird ohne Beschränkung der Allgemeinheit von einem Patienten als Untersuchungsobjekt ausgegangen, wobei es sich meist um einen Menschen handelt. Grundsätzlich kann der Patient aber auch ein Tier sein. Im Folgenden werden daher die beiden Begriffe "Untersuchungsobjekt" und "Patient" auch synonym verwendet. Das Untersuchungsobjekt kann aber auch eine Pflanze oder ein nicht-lebender Gegenstand, z.B. ein historisches Artefakt oder dergleichen sein.

Alternativ kann ein Scanbereich durch manuelle Auswahl einer Start- sowie Endlinie definiert werden, welche mittels eines Lichtvisiers mit Laser-Markierungslinien auf einem Untersuchungsobjekt dargestellt werden, das auf einem in Längsrichtung (z-Richtung) relativ zu einem Scanner (z.B. der Gantry einer CT-Anlage) verfahrbaren Objekttisch (Patientenliege) der bildgebenden Anlage liegt. Die Längsachse des Patienten ist dabei in der Regel parallel zur Längsrichtung des Objekttisches und der Objekttisch befindet sich üblicherweise außerhalb des Scanners. Die Start- und Endlinie erstrecken sich im Wesentlichen in Breitenrichtung (x-Richtung) des Objekttisches, wodurch der Scanbereich in Längsrichtung des Patienten definiert wird. Die Einstellung mittels des Laservisiers ist jedoch relativ zeitaufwendig. Zudem können die Laser-Markierungslinien den Patienten belästigen.

Daneben ist es auch möglich, ein zweidimensionales Bild des auf dem Objekttisch liegenden Untersuchungsobjekts mit einer Kamera aufzunehmen und auf einem Display an einem Steuerungsterminal o. ä. der Anlage darzustellen, wobei in dem Bild die Start- und Endposition des Scanbereichs durch überlagerte Linien dargestellt werden können. Leider ist jedoch die Darstellung eines Start- und Endpunkts des Scanbereichs durch Linien im Bild nicht korrekt. Die Bildentstehung in der Kamera lässt sich nämlich durch das Lochkameramodell annähern, nach dem sich alle Sichtstrahlen in einem Punkt - der Lochblende - schneiden. Jedem Pixel der Kamera ist ein Sichtstrahl zugeordnet, der die Punkte im Raum enthält, die potentiell auf diesen Pixel abgebildet werden. Jeder Pixel im Bild der Kamera entspricht dem Grau- oder Farbwert des Objekts an der Stelle, wo der durch das Pixel und die Lochblende verlaufende Sichtstrahl das Objekt schneidet. Die Sichtstrahlen aller Pixel bilden also ein divergentes Sichtstrahlenbündel.

Dies entspricht nicht der Abbildungsgeometrie des CT-Scans. Der Scanbereich wird in Bewegungsrichtung (z-Richtung) des Objekts durch zwei parallele Ebenen begrenzt. Die zu dieser Ebene korrespondierenden Pixel im Bild der Kamera bilden nur unter ganz bestimmten Bedingungen eine gerade Linie, nämlich im Falle einer telezentrischen Abbildung, d.h. einem (virtuell) unendlichen Abstand der Kamera vom Objekt im LochkameraModell, oder wenn sich die "Lochblende" der Kamera selbst in der anzuzeigenden Raumebene befindet, d.h. direkt über der Start- bzw. Endposition. Eine telezentrische Abbildung ist konstruktiv schwer zu realisieren, da die Eintrittsoptik der Kamera mindestens Objektgröße haben müsste. Eine Kamera, die sich in der anzuzeigenden Raumebene befindet, ist dagegen offensichtlich nur für genau eine Auswahl der Raumebene realisierbar und daher prinzipiell nicht für Start- und Endposition gleichzeitig geeignet.

Das Dokument DE102012201798A1 beschreibt ein Verfahren zur Planung einer Röntgenbildgebung, bei welcher ein bestrahlter Bereich eines Untersuchungsobjekts zur Planung der Untersuchung visualisiert wird. Dabei wird ein Untersuchungsbereich als Repräsentation eines theoretisch bestrahlten Bereichs des Untersuchungsobjekts als schraffierte Fläche einer Repräsentation des Untersuchungsobjekts überlagert. Das Volumen des Untersuchungsobjekts, das bei der Untersuchung durchstrahlt wird, ergibt sich aus der Schnittmenge des Volumens der geplanten Röntgenstrahlung und des Untersuchungsobjekts.

Das Dokument US2015216346A1 offenbart ein Verfahren zum Erzeugen einer personalisierten 3D mesh Darstellung einer Person mittels einer Tiefenbildkamera.

Das Dokument SUMA, EVAN A. ET AL; "Rapid generation of personalized avatars"; IEEE Virtual rality (VR); IEEE; pp. 185; XP032479413; offenbart ein Verfahren zur Erzeugung personalisierter Avatare.

Das Dokument VIVEK, RAJAN ET AL; "A realistic video avatar system for networked virtual environments"; Electronic visualization laboratory University of Illinois at Chicago; US; XP002770331; offenbart ein Verfahren zur Rekonstruktion eines Kopf-Modells anhand von Videoaufnahmen.

Aufgrund der Geometrie des Aufbaus, insbesondere der Tatsache, dass die Kamera einen großen Öffnungswinkel benötigt, um das Untersuchungsobjekt von ihrer Position aus, z.B. an der Zimmerdecke oder an einer Gantry einer CT-Anlage, ganz erfassen zu können, ist der entstehende Fehler nicht unerheblich und eine präzise Einstellung des Scanbereiches mittels eines per 2D-Kamera erfassten Bildes nicht möglich.

Der Fehler in der Liniendarstellung aufgrund des Lochkameramodells lässt sich kompensieren, sofern für jeden abgebildeten Punkt eines Objektes oder einer Szene Tiefeninformation zur Verfügung steht. Tiefeninformation soll dabei den Abstand eines in einem Pixel repräsentierten Objektpunktes von der 2D-Kamera umfassen. Anhand dieser Tiefeninformation kann der Verlauf des Sichtstrahls zwischen Objektpunkt und Lochblende korrigiert und somit der tatsächlich den betrachteten Objektpunkt repräsentierende Pixel ermittelt werden. Dadurch kann perspektivisch korrekt eine Scanbereichsgrenze in einem 2D-Bild dargestellt werden.

Diese Vorgehensweise ist jedoch unflexibel und beschränkt auf im 2D-Bild tatsächlich abgebildete Bestandteile einer Realszene. Komplexe, andersgeartete virtuelle Szenebestandteile, die ggf. durch reelle Szenebestandteile in der Perspektive der 2D-Kamera verdeckt sind, z.B. eine Trajektorie für ein automatisiert bewegbares Operationsinstrument im Rahmen der Interventionsplanung oder reelle Bestandteile der Realszene, die ebenfalls verdeckt sind, z.B. innere, durch die Haut oder Oberfläche des Untersuchungsobjektes verdeckte Organe oder Elemente, lassen sich bislang mit den bekannten Methoden nicht oder nur sehr umständlich in das 2D-Bild integrieren.

Es ist daher eine Aufgabe der vorliegenden Erfindung, alternative Verfahren und Vorrichtung zum perspektivischen Darstellen eines virtuellen, innerhalb einer Realszene angeordneten Szenebestandteils bereit zu stellen, welche besonders flexibel einsetzbar sind und somit die beschriebenen Nachteile überwindet.

Diese Aufgabe wird durch Verfahren und Vorrichtung gemäß den unabhängigen Patentansprüchen gelöst. Weiterbildungen und vorteilhafte Ausgestaltungsvarianten sind jeweils Gegenstand der abhängigen Ansprüche.

Nachstehend wird die erfindungsgemäße Lösung der Aufgabe in Bezug auf das beanspruchte Verfahren als auch in Bezug auf die beanspruchten Vorrichtungen beschrieben. Hierbei erwähnte Merkmale, Vorteile oder alternative Ausführungsformen sind ebenso auch auf die anderen beanspruchten Gegenstände zu übertragen und umgekehrt. Mit anderen Worten können gegenständliche Ansprüche (die beispielsweise auf ein Verfahren gerichtet sind) auch mit Merkmalen, die in Zusammenhang mit einer Vorrichtung beschrieben oder beansprucht sind, weitergebildet sein. Die entsprechenden funktionalen Merkmale des Verfahrens werden dabei durch entsprechende gegenständliche Module oder Einheiten ausgebildet.

Die Erfindung betrifft ein Verfahren zum perspektivischen Darstellen wenigstens eines virtuellen, innerhalb einer Realszene angeordneten Szenebestandteils mittels eines Ergebnisbildes. Das Verfahren umfasst ein
- Erfassen von Tiefenbilddaten der Realszene aus einer ersten Perspektive mittels Tiefenbildsensor,
- Erfassen von 2D-Bilddaten der Realszene aus einer zweiten Perspektive mittels 2D-Kamera,
- Erstellen eines virtuellen, dreidimensionalen Szene-Modells der Realszene unter Berücksichtigung von Tiefeninformation der Tiefenbilddaten,
- Einfügen wenigstens eines virtuellen Szenebestandteils in das dreidimensionale, virtuelle Szene-Modell,
- Erzeugen eines Ergebnisbildes durch perspektivisches Projizieren der 2D-Bilddaten entsprechend der zweiten Perspektive auf das virtuelle, dreidimensionale Szene-Modell umfassend den virtuellen Szene-Bestandteil wobei die Realszene ein auf einer Patientenliege einer medizinischen Bildgebungsanlage befindliches Untersuchungsobjekt umfasst und der wenigstens eine virtuelle Szenebestandteil einer Scanbereichsgrenze, welche in Form einer Grenzkonturlinie ausgebildet ist, entspricht, wobei für den Scanbereich mit der medizinischen Bildgebungsanlage Bilddaten erfasst werden sollen und wobei die Scanbereichsgrenze SBG einer virtuell auf das Untersuchungsobjekt projizierten Licht- oder Laser-Markierung entspricht.

Ein wesentlicher Verfahrensschritt des erfindungsgemäßen Verfahrens ist die Erfassung von Tiefenbilddaten des Untersuchungsobjekts aus einer ersten Perspektive. Ein weiterer wesentlicher Verfahrensschritt ist das Erfassen von 2D-Bilddaten aus einer zweiten Perspektive. Die 2D-Bilddaten umfassen für jeden Pixel dem abgebildeten Objektpunkt entsprechende Farbwerte, z.B. handelt es sich um RGB-Daten, wobei die Attribute R-rot, B-blau und G-grün einen dreidimensionalen Farbraum aufspannen. Die Tiefenbilddaten umfassen zusätzlich zu den Farbwerten auch Abstands- bzw. Tiefeninformation, D-distance, bezüglich des pro Pixel abgebildeten Objektpunktes der Realszene zu der Kamera, z.B. sind die Tiefenbilddaten RGBD-Daten. Andere Datenformate bzw. Farbräume sind denkbar. Insbesondere lassen sich 2D- und 3D-Daten mittels bekannter Verfahren von einer Farbmetrik in eine andere, z.B. YUV, YCbCr oder CMYK, überführen.

Die Erfinder haben nun erkannt, dass in einem weiteren Schritt die Tiefenbilddaten vorteilhaft verwendet werden können, um basierend darauf ein dreidimensionales Szene-Modell der abgebildeten Realszene zu erzeugen. Das Szene-Modell beschreibt eine virtuelle, mathematische Wiedergabe der Realszene, welches die Position, den Verlauf, die Anordnung und/oder Orientierung, etc. der reellen Szene-Bestandteile der Realszene in Bezug zu einem beliebigen Koordinatensystem und zu einander beschreibt.

Unter einer Realszene ist dabei eine Anordnung von beliebigen, reell existierenden Dingen, Gegenständen etc. zueinander zu verstehen, die abgebildet wird. Insbesondere kann die Realszene einem Untersuchungsraum entsprechen, in der sich ein Untersuchungsobjekt auf einem Patiententisch einer medizinischen Bildgebungsanlage befindet. Dies entspricht, wie weiter unten näher erläutert wird, dem Hauptanwendungsfall der vorliegenden Erfindung.

In dieses virtuelle Szene-Modell lassen sich nun erfindungsgemäß in einfacher Weise beliebig geartete, insbesondere dreidimensionale und beliebig viele virtuelle Szenebestandteile einfügen. Ein virtueller Szenebestandteil kann jedes beliebige, nicht tatsächlich in der Realszene enthaltene und damit nicht mittels Tiefenbildsensor und/oder 2D-Kamera abgebildete Objekt oder Element sein. Die Art, seine Eigenschaften sowie Informationen bezüglich seiner Position, Anordnung, Orientierung in besagtem Szene-Modell, etc. können z.B. durch einen Benutzer vorab festgelegt oder in Abhängigkeit einer im Anschluss geplanten Untersuchung, ggf. unter Berücksichtigung von Patientendaten, insbesondere Informationen zu seiner Anatomie, automatisch vorgeschlagen und dem Nutzer zur Bestätigung angezeigt oder ohne User-Input festgelegt werden. Aus Benutzerangaben kann derart z.B. eine mathematische Beschreibung des virtuellen Szenebestandteils bestimmt werden, die dann unter Berücksichtigung von Referenzangaben in Bezug zu anderen in dem virtuellen Szene-Modell enthaltenen Szene-Bestandteilen in das virtuelle Szene-Modell eingefügt werden kann.

Das Ergebnisbild umfassend die perspektivische Darstellung des wenigstens einen virtuellen Szenebestandteils ergibt sich, indem die 2D-Bilddaten perspektivisch entsprechend der zweiten Perspektive auf das virtuelle, dreidimensionale Szene-Modell umfassend den virtuellen Szene-Bestandteil projiziert werden. Dadurch wird das dreidimensionale, virtuelle Szene-Modell samt eingefügtem, virtuellem Szenebestandteil in die Perspektive der 2D-Kamera überführt, sodass der virtuelle Szenebestandteil korrekt in der Perspektive der 2D-Bilddaten dargestellt wird. Für den Betrachter ergibt sich so vorteilhaft eine Darstellung der Realszene, die dem Bildeindruck des 2D-Kamera-Bildes entspricht jedoch mitsamt den entsprechenden, perspektivisch korrekten, auch auf den eingefügten, virtuellen Szenebestandteil wirkenden Verzerrungen.

Die Erfindung beruht also auf der Abkehr davon, einen virtuellen Szenebestandteil unter Berücksichtigung von Tiefeninformation in eine Perspektive von erfassten 2D-Bilddaten zu überführen, hin zur virtuellen Modellierung der gesamten Realszene, in welches virtuelle Szenebestandteile auf einfache Weise integriert werden können, um dann das gesamte Modell samt virtuellem Bestandteil aus der 2D-Perspektive darzustellen.

Derart erfolgt eine korrekte, für den Anwender verständliche und aufgrund der Perspektivenwahl insbesondere gewohnte und Realszenen-äquivalente Darstellung einer Positionsplanung. Eine zusätzlich belastende Strahlenabgabe an den Patienten für die Untersuchungsplanung entfällt, genauso wie ein mühsames Hin- und Herfahren der Patientenliege. Die Untersuchungszeit kann für den Patienten minimiert werden, da die Verweildauer des Patienten in der Bildgebungsanlage minimiert wird. Die Vorrichtung wird günstiger in Hinblick auf Herstellung, Betrieb, Wartung und Reparatur, da Komponenten ganz entfallen können oder weniger stark genutzt werden. Zudem ist diese Vorgehensweise durch eine besonders hohe Flexibilität gekennzeichnet, denn 1) erlaubt die erfindungsgemäße Vorgehensweise die Betrachtung der Realszene samt eingefügtem virtuellem Szene-Bestandteil aus einer beliebigen, dritten Perspektive und 2) kann können beliebig viele und beliebig geartete virtuelle Szene-Bestandteile perspektivisch korrekt, d.h. unter Berücksichtigung von ggf. perspektivisch bedingten Verdeckungen von Realszene-Bestandteilen dargestellt werden.

Gemäß einer ersten, bevorzugten Ausführungsform der Erfindung erfolgt das perspektivische Projizieren mittels eines Renderers.

Unter einem Renderer ist dabei eine Render-Software bzw. eine Render-Pipeline zu verstehen, die dazu dient, eine Darstellung bzw. Visualisierung von dreidimensionalen Körpern bzw. Objekten zu erzeugen. Ausgangspunkt eines Volumen-Renderers ist ein dreidimensionales, virtuelles Modell der zu rendernden Körper oder Objekte. Typischerweise handelt es sich um ein virtuelles Oberflächen- oder Volumenmodell. Ein Oberflächenmodell, z.B. in Form eines Gitters beschreibt Grenz- bzw. Oberflächen der Körper oder Objekte der abzubildenden Realszene, wohingegen ein Volumenmodell auch physikalische Eigenschaften wie Elastizität, Dichte oder dergleichen der abgebildeten Körper oder Objekte beschreiben kann. Die Volumeninformation lässt sich insbesondere mittels Volumenelementen in Form von Voxeln beschreiben. Werte für einzelne Volumenelemente liegen häufig in der Form von sog. Grauwerten vor.

Die Voxelwerte werden allgemein z.B. durch eine medizinische Bildgebungsanlage gewonnen. Typisch sind z.B. Messungen mittels Computertomographie (CT), welche für verschiedene Aufnahmepositionen Projektionen liefern, aus welchen die Voxelwerte rekonstruiert werden. Andere Möglichkeiten sind Ultraschallverfahren oder Kernspinaufnahmen (MRT). Die Voxelwerte liegen dann üblicherweise in der Form von sog. Grauwerten vor, welche ein Maß für die jeweilige Dichte des Objekts an diesem Ort darstellen.

Mittels Volume Rendering wird aus den Voxelwerten eine dreidimensionale Darstellung eines Objekts bzw. Körpers auf einer zweidimensionalen Darstellungsfläche (z.B. Bildschirm einer medizinischen Bildgebungsanlage) erzeugt. Dabei werden aus den Voxeln sog. Pixel erzeugt (ggf. mit der Zwischenstufe von aus den Voxeln durch Interpolation gewonnenen Objektpunkten), aus welchen das Bild der zweidimensionalen Bildanzeige zusammengesetzt ist. Um auf einer zweidimensionalen Anzeige drei Dimensionen zu visualisieren, wird in der Regel ein sogenanntes Alpha-Compositing bzw. eine Alpha-Zerlegung vorgenommen. Bei dieser Standardmethode werden Voxeln bzw. aus Voxeln gebildeten Volumenpunkten sowohl Farben als auch Durchlässigkeitswerte, genauer gesagt Werte für die Undurchlässigkeit bzw. Opazität (üblicherweise bezeichnet mit dem englischen Begriff Opacity, der die Durchlässigkeit bzw. die Deckkraft verschiedener Schichten des Körpers ausdrückt) zugeordnet. Konkreter werden einem Objektpunkt z.B. drei Farben in Form eines Drei-Tupels, der die Anteile der Farben rot, grün und blau kodiert (sog. RGB-Wert), und ein sog. Alpha-Wert, der die Undurchlässigkeit parametrisiert, zugeordnet. Zusammen bilden diese Größen einen Farbwert RGBA, der mit den Farbwerten anderer Objektpunkte zu einem Farbwert für das Pixel kombiniert bzw. gemischt werden kann (für die Visualisierung von teilweise transparenten Objekten üblicherweise mittels eines sog. Alpha Blending).

Für die Zuordnung eines passenden Farbwertes wird meist mit einem Beleuchtungsmodell gearbeitet. Dieses Beleuchtungsmodell berücksichtigt Lichteffekte (in der Regel Reflexionen des Lichtes an Oberflächen des Objektes; dabei kann es sich um die äußere Oberfläche oder um Oberflächen innerer Schichten des Objektes handeln) bei einer zum Zwecke der Visualisierung modellierten bzw. simulierten Bestrahlung des Objektes.

Es gibt in der Literatur eine Reihe von Beleuchtungsmodellen, die angewandt werden. Gebräuchlich ist z.B. das Phong- oder Blinn-Phong-Modell.

Eines der meistbenutzten Verfahren zum Volume Rendering ist das sog. Ray-Casting bzw. die Simulation einer Lichteinstrahlung zur Darstellung bzw. Visualisierung des Körpers.

Beim Ray-Casting werden imaginäre Strahlen, die vom Auge eines imaginären Betrachters ausgehen (hier spricht man auch von einer virtuellen Renderer-Kamera), durch einen Körper bzw. ein Objekt gesendet. Entlang der Strahlen werden für Abtastpunkte RGBA-Werte aus den Voxeln bestimmt und zu Pixeln für ein zweidimensionales Bild mittels Alpha Compositing bzw. Alpha Blending vereinigt. Beleuchtungseffekte werden dabei üblicherweise mittels eines der oben abgesprochenen Beleuchtungsmodelle im Rahmen eines mit "Shading" bezeichneten Verfahrens berücksichtigt.

Entsprechend werden erfindungsgemäß die 2D-Bilddaten entlang der Sichtstrahlen der 2D-Kamera auf das Szene-Modell perspektivisch projiziert. Damit wird für das virtuelle Szene-Modell die zweite Perspektive der 2D-Kamera eingenommen. Mit anderen Worten wird die virtuelle Renderer-Kamera in derselben Pose angeordnet wie die 2D-Kamera, sodass aus Blickrichtung der 2D-Kamera eine realistische Darstellung der Realszene umfassend den wenigstens einen virtuellen Szene-Bestandteil berechnet und angezeigt werden kann.

Diese Vorgehensweise basiert einerseits auf der Erkenntnis, dass Render-Software zumindest im Bereich der Augmented Reality sehr weit verbreitet ist und insbesondere, eigene, inhouse entwickelte Rendering-Pipelines, wie z.B. das Volume-Rendering(VRT)-Feature von Siemens syngo, für die Ausführung des erfindungsgemäßen Verfahrens zur Verfügung stehen und kostenneutral zur Realisierung der Erfindung eingesetzt werden können. Die Erfindung greift folglich auf bestehende, technische Lösungsansätze zurück. Zum anderen haben die Erfinder erkannt, dass sich die existierenden algorithmischen Ansätze aus dem Bereich der Augmented Reality unter Zuhilfenahme der Tiefeninformation auf das der Erfindung zugrunde liegende technische Problem der perspektivisch korrekten Integration von virtuellen Szenebestandteilen in eine 2D-Darstellung übertragen lassen. Zum anderen ermöglicht der Einsatz von Render-Software neben dem Wechsel der Betrachtungsrichtung auch vorteilhaft eine Manipulation, z.B. ein Einfärben oder Clipping oder dergleichen, von in dem virtuellen Szene-Modell enthaltener Bildinformation bezüglich der dargestellten Realszene. Somit kann der perspektivisch korrekten Darstellung je nach Anwendungsfall ein erhöhter Informationsgehalt zukommen.

Gemäß einer anderen bevorzugten Ausführungsvariante erfolgt das perspektivische Projizieren unter Berücksichtigung extrinsischer und/oder intrinsischer Kamera-Parameter der 2D-Kamera und/oder des Tiefenbildsensors. Sowohl extrinsische als auch intrinsische Kamera-Parameter ergeben sich jeweils aus einer Kalibrierung einer Kamera. Bei der intrinsischen Kalibrierung wird die interne Geometrie der Kamera bestimmt, beispielsweise die Brennweite der Kamera. Weiterhin können bei der intrinsischen Kalibrierung Korrekturfunktionen für optische Fehler wie Aberrationen bestimmt werden. Weiter können bei der intrinsischen Kalibrierung die Zentrierung des Sensors, Verkippungen optischer Elemente der Kamera und/oder andere Abbildungsfehler berücksichtigt werden. Die derart gewonnenen intrinsischen Kamera-Parameter können dann herangezogen werden, um die erfassten Bilddaten zu korrigieren.

Bei der extrinsischen Kalibrierung wird die Pose einer Kamera in Raumkoordinaten bestimmt. Solche Raumkoordinaten werden auch als externe Koordinaten oder Weltkoordinaten bezeichnet. Bei der Pose handelt es sich um die Kombination von Orientierung und Position. Daher kann die Pose durch eine Drehmatrix und einen Ortsvektor beschrieben werden. Zusätzlich kann die extrinsische Kalibrierung auch Lageunterschiede zwischen Farb- und Tiefensensor des Tiefenbildsensors umfassen.

Zur intrinsischen sowie zur extrinsischen Kalibrierung muss jeweils ein Gleichungssystem gelöst werden. Daher müssen bekannte Informationen bereitgestellt werden um die unbekannten Parameter des Gleichungssystems zu bestimmen. Bei den unbekannten Parametern handelt es sich beispielsweise um die Brennweite oder um einen Eintrag in der Drehmatrix.

Während eine intrinsische Kalibrierung zur Bestimmung von Kamera-Parametern in der Regel nur ein einziges Mal für eine Kamera durchgeführt werden muss, muss eine extrinsische Kalibrierung oft mehrmals während der Betriebsdauer einer Kamera durchgeführt werden, beispielsweise immer dann, wenn sich die Pose der Kamera ändert.

Hier einsetzbare Kalibrierungsverfahren sind dem Fachmann bekannt und werden daher nicht weiter erläutert.

Um eine korrekte Projektion von 2D-Bilddaten auf das virtuelle Szene-Modell vornehmen zu können, müssen extrinsische Kamera-Parameter von 2D-Kamera und Tiefenbildsensor bekannt sein. Die Kalibrierung kann dazu entweder in Bezug auf ein globales Koordinatensystem, insbesondere ein Koordinatensystem einer medizinischen Bildgebungsanlage oder aber direkt zwischen 2D-Kamera und Tiefenbildsensor erfolgen. Die Kalibrierung kann im Rahmen der Erfindung zur Bestimmung der Kamera-Parameter durchgeführt werden oder die Kamera-Parameter können als bekannt vorausgesetzt werden.

Eine besonders einfache Ausgestaltungform der Erfindung ergibt sich, wenn die 2D-Bilddaten und die Tiefenbilddaten aus der gleichen bzw. aus annähernd der gleichen Perspektive erfasst werden. Mit anderen Worten unterscheiden sich erste und zweite Perspektive gar nicht oder nur unmerklich. Dies hat den Vorteil, dass der Projektionsschritt des erfindungsgemäßen Verfahrens besonders einfach ausfallen kann, da nun ggf. lediglich intrinsische Kamera-Parameter dabei berücksichtigt werden müssen. Zudem ermöglicht diese Vorgehensweise die Aufnahme von 2D-Bilddaten und Tiefenbilddaten mit nur einer Kamera, die 2D-Bilddaten entsprechen dann z.B. den Farbdaten des Farbsensors des Tiefenbildsensors.

In einem Beispiel der Erfindung werden die 2D-Bilddaten sowie die Tiefenbilddaten zeitgleich oder quasi-zeitgleich (d.h. etwa zeitgleich, mit nur geringem zeitlichen Abstand, z.B. von max. 1 s oder weniger) erfasst. So können sich unerwünschte Bewegungen innerhalb der Realszene nicht in den Bilddaten niederschlagen und zu einem fehlerhaften Zusammenhang zwischen 2D-Bilddaten und Tiefenbilddaten führen.

Gemäß einer bevorzugten Ausführungsform des erfindungsgemäßen Verfahrens handelt es sich bei dem virtuellen, dreidimensionalen Szene-Modell um ein Oberflächenmodell. Dieses ist dadurch gekennzeichnet, dass es nur unter Verwendung der Tiefenbilddaten entsteht. Es repräsentiert entsprechend alle Grenz- bzw. Oberflächen der in der Realszene abgebildeten Objekte, Körper, Elemente, etc. Dieses Modell umfasst insbesondere keine Information bezüglich einer Beschaffenheit, der inneren Struktur und/oder physikalischen Eigenschaften von Objekten in der abgebildeten Realszene. Das Modell beschreibt die Grenz- bzw. Oberflächen mathematisch. Dabei werden die Tiefenbilddaten bevorzugt einer Polygonisierung unterzogen, um eine bestmögliche Beschreibung der abgebildeten Oberflächen zu erhalten. Mögliche Polygonisierungsalgorithmen sind z.B. eine Triangulierung, bei der Oberflächen mittels aneinander gereihter Dreiecke nachgebildet werden. Diese Methode eignet sich deshalb besonders gut, weil sie häufig auch von Render-Software genutzt wird. Andere Modellbescheibungen, z.B. ein Cutting Cube Algorithmus, sind ebenfalls möglich. Alternativ kann die Oberfläche auch durch modellhafte Beschreibung mittels Freiformflächen angenähert werden. Die Beschränkung auf die in den Tiefenbilddaten enthaltenen Informationen bezüglich der in der Realszene abgebildeten Oberflächen ist für den hiesigen Hauptanwendungsfall, der perspektivisch korrekten Darstellung von Scanbereichsbegrenzungslinien vollkommen ausreichend, da in diesem Fall lediglich die Perspektive der 2D-Kamera mittels Render-Software nachgebildet und in diesem Sinne ,eingenommen' werden muss.

Die Erfindung ist jedoch, wie oben bereits erwähnt, nicht auf Szene-Modelle in Form von Oberflächenmodellen beschränkt. Insbesondere können sich aus anderen Datenquellen zusätzliche Informationen über die Beschaffenheit, innere Struktur, etc. von Objekten in der Realszene ergeben und das Szene-Modell anreichern. Z.B. können vorhandene Bilddaten des Untersuchungsobjektes aus vorherigen Untersuchungen durch eine medizinische Bildgebungseinheit vorhanden sein, insbesondere Bilddaten in Form von CT-Aufnahmen, MRT-Aufnahmen, etc. Diese Bilddaten weisen entweder einen bekannten, geometrischen Bezug zu dem Szene-Modell auf oder dieser lässt sich mittels bekannter Registrierungsverfahren herstellen, sodass die Bildinformation, insbesondere Information bezüglich Beschaffenheit, innerer Struktur und/oder physikalischen Eigenschaften, aus den Bilddaten in das Szene-Modell integriert werden kann.

Wie bereits erwähnt, umfasst die Realszene gemäß einer besonders bevorzugten Ausführungsform der Erfindung ein auf einer Patientenliege einer medizinischen Bildgebungsanlage befindliches Untersuchungsobjekt und der wenigstens eine virtuelle Szenebestandteil entspricht einer Scanbereichsgrenze für einen Scanbereichs, für welchen mittels medizinischer Bildgebungsanlage Bilddaten erfasst werden sollen. Dabei sei auch die Aufnahme eines Topogramms mit abgedeckt, auch wenn für ein Topogramm kein "Scan" im klassischen Sinne erfolgt. Entsprechend kann erfindungsgemäß auch eine Topogrammbegrenzung erfolgen. Die Scanbereichsgrenze kann vorzugsweise als Grenzkonturlinie ausgebildet sein, die einer beliebig gekrümmten Kurve entlang der Oberfläche der abgebildeten Realszene verläuft. Insbesondere verläuft die Grenzkonturlinie quer zu einer Bewegungsrichtung eines Objekttisches, auf dem das Untersuchungsobjekt angeordnet ist, oder, für den Fall von Topogrammen in einer beliebigen von der senkrechten zur Bewegungsrichtung des Untersuchungsobjektes abweichenden Ebene. Die Scanbereichsgrenze kann aber auch als Scanbereichsbegrenzungsebene in beliebiger Lage ausgebildet sein.

Erfindungsgemäß ist eine sehr präzise Festlegung eines Scanbereichs möglich, da der Verlauf einer Scanbereichsgrenze anhand des Szene-Modells realitätsnah nachgebildet werden kann.

Selbstredend können mehr als ein virtueller Szenebestandteil in das Szene-Modell eingeführt werden. In einem Beispiel der Erfindung können virtuelle Szenebestandteile in Form von zumindest zwei Grenzkonturlinien und/oder Grenzlinien eines Scanbereichs in Form einer Startlinie bzw. einer Endlinie eingefügt werden, zwischen denen der Scanbereich definiert ist. Start- und Endlinie werden dann wenigstens innerhalb der seitlichen Außenumrisse des Patienten dargestellt, können aber auch darüber hinaus verlaufen, insbesondere über die gesamte Breite des 2D-Bildes. Alternativ können andere virtuelle Szenebestandteile integriert werden, so z.B. eine oder mehrere Trajektorien für Interventionsinstrumente oder dergleichen.

Hierbei erweist sich das erfindungsgemäße Verfahren als besonders vorteilhaft, da das virtuelle Szene-Modell nun neben einer Betrachtung aus der dem 2D-Bild entsprechenden, zweiten Perspektive (Hauptanwendungsfall der Erfindung) auch beliebige weitere Betrachtungsrichtungen zulässt, sodass der Betrachter einen allumfassenden Gesamteindruck der Anordnung/Position des virtuellen Szenebestandteils relativ zur Realszene erlangen kann. Darüber hinaus lässt sich das Szene-Modell neben dem virtuellen Szene-Bestandteil mit weiterer Bildinformation anreichern, z.B. lassen sich innere Organe eines Patienten oder innere, in dem 2D-Bild verdeckte Bestandteile der Realszene, jeweils entsprechend zusätzlicher Szenebestandteile beliebiger Herkunft, z.B. aus vorherigen Aufnahmen mit einer medizinischen Bildgebungsanlage, auf einfache Weise in das Modell integrieren.

Erfindungsgemäß entspricht die Scanbereichsgrenze einer virtuell auf das Untersuchungsobjekt projizierten Licht- oder Laser-Markierung. Dadurch kann erfindungsgemäß dem Nutzer der Bildgebungsanlage ein Bildeindruck vermittelt werden, den er von bisherigen Einstellungen des Scanbereichs gewohnt ist. Durch die Umsetzung der Erfindung mittels eines Renderers können daneben beliebige Belichtungs- oder anders geartete, visuelle Effekte konstruiert oder realistisch nachgebildet werden.

Die vorliegende Erfindung ermöglicht durch den Einsatz einer Render-Software insbesondere auch, dass Manipulationen (Perspektivenwechsel, Einfärbungen, Clippings, etc.) durchgeführt werden können. Dabei wird eine realistische Darstellung erreicht, indem bei der Berechnung zwei wesentliche Effekte unterschieden werden. Der erste Effekt ist die Schattenbildung (Shadows). Dieser Effekt rührt daher, dass für einen Punkt auf einem simulierten Strahl durch ein Objekt der Pfad zwischen dem objektpunkt und der virtuellen Lichtquelle bzw. den Lichtquellen Hindernisse aufweist, d.h. der Lichtstrahl unter Umständen nicht ungehindert den Objektpunkt erreichen kann. Die konzeptionell einfachste Methode zur Berechnung dieses Schatteneinflusses ist von dem betrachteten Punkt aus einen Strahl Richtung Lichtquelle zu schicken (bzw. Strahlen zu den verschiedenen Lichtquellen zu schicken), um so zu ermitteln, welche Anteile des Lichtes zu dem Punkt durchdringen können.

Davon zu unterscheiden ist der Effekt von Streulicht (in der Fachliteratur ist der Ausdruck "ambient occlusion" bzw. Umgebungsverdeckung üblich). Dieser Effekt ist auf Streulicht zurückzuführen und daher sehr wichtig, weil er Strukturen sichtbar macht, die allein durch das direkte Licht nicht sichtbar werden. Generell führt die Lichtstreuung erst einmal dazu, dass auf ein Objekt Licht aus allen Richtungen trifft. Dieses Streulicht kann durch Materie absorbiert werden, so dass sich eine ungleichmäßige Erhellung des Objekts durch den Streulichtanteil ergibt. Ein typisches Beispiel für diesen Effekt sind die Ecken eines Zimmers, welche als dunkler erscheinen als dessen Zentrum. Die Absorption vom Streulicht wird z.B. mit derselben Methode untersucht, mit der auch die Schattenbildung ermittelt wird. Da das Streulicht aber von keiner festen Quelle, sondern von allen Richtungen kommt, werden Strahlen in stochastischer Weise von einer Hemisphäre, die lokal mit einer Oberfläche übereinstimmt, gesendet, und so überprüft, wie viel Streulicht absorbiert wird. Man spricht bei dem Versenden von Strahlen zur Ermittlung von Beleuchtungseigenschaften von Ray-Tracing, und im Fall von stochastischer Strahlabtastung mittels "ambient occlusion" von Monte Carlo Ray-Tracing.

Gemäß einer weiteren Ausgestaltungsform des erfindungsgemäßen Verfahrens erfolgt das Einfügen des virtuellen Szene-Bestandteils in das virtuelle, dreidimensionale Modell anhand von Benutzereingaben. Dies ermöglicht eine besonders flexible Anpassbarkeit des virtuellen Szenebestandteils nach den Vorstellungen des Nutzers.

Dabei kann es gemäß einer weiteren Ausgestaltungsform der Erfindung besonders von Vorteil sein, wenn der Benutzer die Möglichkeit erhält, die Anordnung des virtuellen Szenebestandteils unter Verwendung der 2D-Bilddaten zu ermitteln. Gemeint sei hiermit die Anordnung des virtuellen Szenebestandteils in dem dreidimensionalen Szene-Modell. Dazu kann vorgesehen sein, dass dem Nutzer die 2D-Bilddaten z.B. über eine Anzeigevorrichtung, z.B. einen LCD-Display angezeigt werden und diese mittels einer Eingabevorrichtung, z.B. eine Maus und dazu gehörigen Cursor oder ein berührungsempfindliches Display, manipuliert werden können. Das entspricht in großen Teilen vorteilhaft dem für den Nutzer bislang bekannten Vorgehen.

Gemäß einer anderen Ausführungsform der Erfindung werden die 2D-Bilddaten des Untersuchungsobjekts im sichtbaren Wellenlängenbereich erfasst, also z.B. zwischen 380 nm und 780 nm. Besonders bevorzugt wird das 2D-Bild als Farbbild sichtbar dargestellt. Alternativ ist eine Darstellung als Graustufenbild (mit z.B. 256 Stufen) ebenfalls denkbar. Die Aufnahme der 2D-Bilddaten im Infrarot-Bereich, z.B. zwischen 780 nm und 1000 nm, ist jedoch auch möglich.

Gemäß einer bevorzugten Ausführungsform des erfindungsgemäßen Verfahrens werden die Tiefenbilddaten des Untersuchungsobjekts im Infrarot-Wellenlängenbereich erfasst, z.B. zwischen 780 nm und 1000 nm. Dadurch wird eine besonders robuste Ermittlung der Tiefenbilddaten ermöglicht. Andere Wellenlängenbereiche zur Ermittlung der Tiefenbilddaten sind ebenfalls möglich.

Die Erfindung betrifft ferner eine Vorrichtung zum perspektivischen Darstellen eines virtuellen, innerhalb einer Realszene angeordneten Szenebestandteils mittels eines Ergebnisbildes, umfassend
- einen Tiefenbildsensor, welcher Tiefenbilddaten der Realszene aus einer ersten Perspektive erfasst,
- eine 2D-Kamera, welche 2D-Bilddaten der Realszene aus einer zweiten Perspektive erfasst,
- eine Modell-Erstellungseinheit, die ein virtuelles, dreidimensionales Szene-Modell der Realszene unter Berücksichtigung von Tiefeninformation der Tiefenbilddaten erstellt,
- eine Einfüge-Einheit, die einen virtuellen Szenebestandteil in das dreidimensionale, virtuelle Szene-Modell einfügt,
- eine Erzeugungs-Einheit, die ein Ergebnisbild durch perspektivisches Projizieren der 2D-Bilddaten entsprechend der zweiten Perspektive auf das virtuelle, dreidimensionale Szene-Modell umfassend den virtuellen Szene-Bestandteil erzeugt,
wobei die zu erfassende Realszene ein auf einer Patientenliege einer medizinischen Bildgebungsanlage befindliches Untersuchungsobjekt umfasst und der wenigstens eine virtuelle Szenebestandteil einer Scanbereichsgrenze, welche in Form einer Grenzkonturlinie ausgebildet ist, entspricht, wobei für den Scanbereich mit der medizinischen Bildgebungsanlage Bilddaten erfasst werden sollen und wobei die Scanbereichsgrenze einer virtuell auf das Untersuchungsobjekt projizierten Licht- oder Laser-Markierung entspricht.

Der Tiefenbildsensor kann insbesondere bevorzugt als TOF-Kamera ("Time Of Flight"-Kamera), z.B. mit einem Photonenmischdetektor (PMD) ausgebildet sein, welche mittels Laufzeitverfahren mit optisch sichtbarem Licht oder InfrarotLicht arbeitet. Solche Einheiten sind relativ kostengünstig. Alternativ kann der Sensor die 3D-Daten auch durch ein Stereobild-Verfahren oder durch Beleuchtung mit strukturiertem Licht oder eine Kombination daraus verfügbar machen. Bei der 2D-Kamera kann es sich um eine Foto-Kamera oder um eine Video-Kamera handeln, welche 2D-Bilddaten der Realszene erstellt.

Die Modell-Erstellungseinheit und/oder die Einfüge-Einheit und/oder die Erzeugungseinheit können z.B. in Form von Software oder Softwaremodul, beispielsweise in einer Steuerungseinheit einer medizinischen Bildgebungsanlage ausgebildet und integriert sein, wie später noch genauer erläutert wird. Die einzelnen Einheiten können in einer physikalischen Einheit zusammengefasst sein oder als separate Einheiten, insbesondere auch dezentral, ausgebildet sein. Jedenfalls stehen sie in Datenverbindung (kabellos oder kabelgebunden) zu einander, um die für die jeweiligen Prozessschritte notwendigen Daten austauschen zu können. Insbesondere die Einfüge-Einheit und/oder die Erzeugungs-Einheit können Bestandteil einer bereits existierenden Render-Pipeline sein, welche typischerweise als Hardware realisiert ist.

In einer bevorzugten Ausführungsform der Erfindung sind die 2D-Kamera und der Tiefenbilddatensensor zu einer Kamera-Einheit zusammengefasst. Dies ermöglicht die Aufnahme von Bilddaten in 2D und 3D aus der gleichen oder annähernd der gleichen Perspektive. Dabei bedeutet Kamera-Einheit, dass zumindest beide Kameras in einem Gehäuse angeordnet sind. Einerseits kann vorgesehen sein, dass die optischen Achsen der 2D-Bild-Kamera und des Tiefenbildsensors bevorzugt eine maximale Abweichung von ca. ± 1% einer maximalen Abmessung des Scanbereiches aufweisen. Hat der Scanbereich z.B. eine Länge in z-Richtung von 0,5 m, dann beträgt die maximale Abweichung der optischen Achsen der 2D-Bild-Kamera und des Tiefenbildsensors ca. 5 mm. Bevorzugt liegt die Entfernung zwischen der Kamera-Einheit und einem in der Realszene befindlichen Untersuchungsobjekt bzw. dessen geometrischer Mitte bei z.B. 2 m ± 50%, also zwischen 1 m und 3 m. Hiermit kann das Risiko reduziert werden, dass die Tiefenbilddaten des Untersuchungsobjekts Fehldaten auf Grund von Eigenabschattungen des Untersuchungsobjekts aufweist. Damit kann eine vollständige Ermittlung des Scanbereichs ohne ggfs. verfälschende Inter- oder Extrapolationen der Tiefenbilddaten erfolgen. Zudem wird damit einfacher verhindert, dass die Auflösung und damit die Anzahl der Pixel insbesondere in den Tiefenbilddaten zu gering ist. Alternativ sind die 2D-Kamera und die Tiefenbilddatensensor nicht nur räumlich, sondern auch strukturell und logisch zusammengefasst. Die Kamera-Einheit ist hierbei insbesondere als 3D-Kamera in einer der oben beschriebenen Varianten ausgebildet und dazu eingerichtet, neben den Tiefenbilddaten auch 2D-Bilddaten zu erzeugen, wobei sich die 2D-Bilddaten z.B. als Detektordaten des Farb-Sensors ergeben können.

Die Kamera-Einheit kann z.B. einfach an eine Zimmerdecke oder an einen Rahmen einer medizinischen Bildgebungsanlage montiert und mit einer Steuerungseinheit der Anlage signalleitend (drahtgebunden oder drahtlos) gekoppelt werden.

Die Erfindung betrifft daneben auch eine medizinische Bildgebungsanlage, insbesondere ein Computertomographiegerät, umfassend eine erfindungsgemäße Vorrichtung.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugszeichen versehen. Die Figuren sind in der Regel nicht maßstäblich. Es zeigen:
- Figur 1: eine medizinische Bildgebungsanlage in Form einer Computertomographie-Vorrichtung gemäß einem Ausführungsbeispiel der Erfindung,
- Figur 2: ein Ablaufschema des erfindungsgemäßen Verfahrens gemäß einem Ausführungsbeispiel der Erfindung, und
- Figur 3a - 3f: das erfindungsgemäße Verfahren in einem Ausführungsbeispiel anhand von erfassten Bilddaten eines Untersuchungsobjektes und Szene-Modell-Ansichten des Untersuchungsobjektes sowie ein sich erfindungsgemäß ergebendes Ergebnisbild.

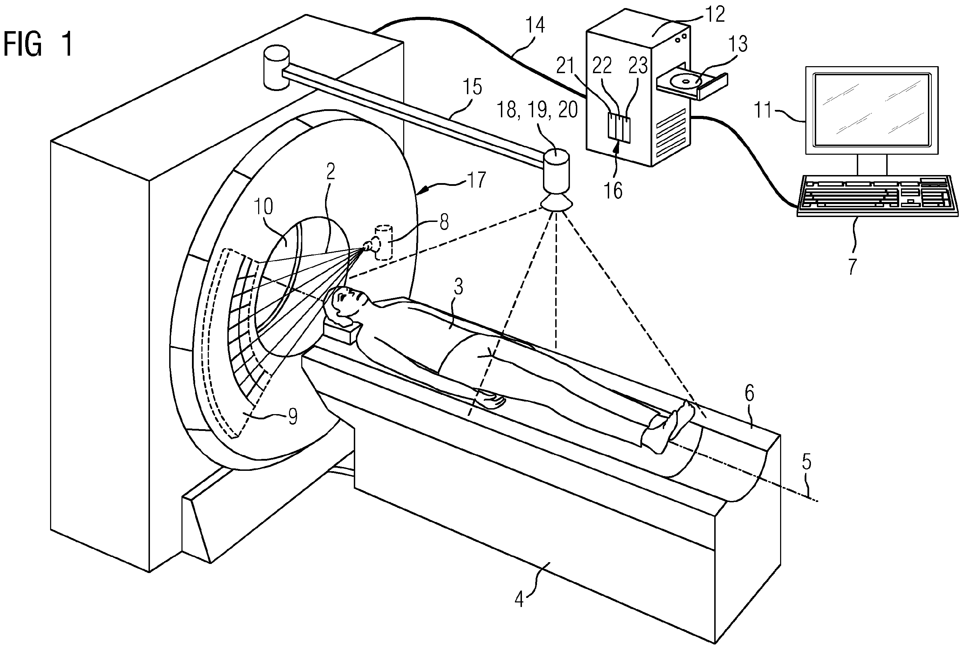

**Figur 1** zeigt eine medizinische Bildgebungsanlage in Form einer Computertomographie-Vorrichtung. Der hier gezeigte Computertomograph verfügt über eine Aufnahmeeinheit 17, umfassend eine Strahlungsquelle 8 sowie einen Strahlungsdetektor 9. Die Aufnahmeeinheit 17 rotiert während der Aufnahme von Röntgenprojektionen um eine Systemachse 5, und die Röntgenquelle emittiert während der Aufnahme Röntgenstrahlen 2.

Ein Patient 3 liegt bei der Aufnahme von Röntgenprojektionen auf einer Patientenliege 6. Die Patientenliege 6 ist so mit einem Liegensockel 4 verbunden, dass er die Patientenliege 6 mit dem Patienten 3 trägt. Die Patientenliege 6 ist dazu ausgelegt, den Patienten 3 entlang einer Aufnahmerichtung durch die Öffnung 10 der Aufnahmeeinheit 17 zu bewegen. Die Aufnahmerichtung ist in der Regel durch die Systemachse 5 gegeben, um die die Aufnahmeeinheit 17 bei der Aufnahme von Röntgenprojektionen rotiert. In diesem Beispiel ist die Körperachse des Patienten gleich der Systemachse 5. Beide Achsen liegen auf der Z-Achse eines dreidimensionalen, kartesischen Koordinatensystems (nicht dargestellt). Bei einer Spiral-Aufnahme wird die Patientenliege 6 kontinuierlich durch die Öffnung 10 bewegt, während die Aufnahmeeinheit 17 um den Patienten 3 rotiert und Röntgenprojektionen aufnimmt. Damit beschreiben die Röntgenstrahlen auf der Oberfläche des Patienten 3 eine Spirale.

Die Röntgenaufnahmevorrichtung verfügt über eine Kamera-Einheit 18. Diese umfasst in einem gemeinsamen Gehäuse eine 2D-Kamera, die eine zweidimensionale Darstellung der abgebildeten Szene umfassend den Patienten 3 in Graustufen oder Farbwerten erzeugt, und eine 3D-Kamera bzw. einen Tiefenbildsensor, beispielsweise in Form eine Stereokamera, Timeof-Flight Kamera oder eines interferometrischen Systems oder dergleichen, und umfasst die für die jeweilige Aufnahmetechnik notwendigen Komponenten wie geeignete Lichtquellen und Detektionseinheiten. Die Kamera-Einheit 18 ist in diesem Beispiel oberhalb der Patientenliege 6 angeordnet und über eine Haltevorrichtung 15 fest mit der Bildaufnahmevorrichtung verbunden. Die Kamera-Einheit 18 kann auch an der drehbaren Aufnahmeeinheit 17 befestigt sein. Insbesondere bei einer kippbaren drehbaren Aufnahmeeinheit 17 kippt die Kamera-Einheit 18 selbstredend mit. Alternativ ist die Kamera-Einheit 18 mit freiem Blick auf den Patienten 3 stationär oder mobil im Untersuchungsraum angeordnet, beispielsweise an der Decke des Untersuchungsraumes, oder sie ist an einem Stativ angeordnet, welches im Untersuchungsraum frei bewegbar ist.

Die Realszene entspricht in diesem Ausführungsbeispiel dem Untersuchungsraum, umfassend den Patienten 3 auf der Patientenliege 6. Da sowohl die 2D-Kamera als auch der Tiefenbildsensor in einer Kamera-Einheit zusammengefasst sind, sind in diesem Ausführungsbeispiel die erste und die zweite Perspektive der Kameras gleich bzw. annähernd identisch.

Das Sichtfeld der Kamera-Einheit 18 ist in diesem Ausführungsbeispiel so groß, dass es den Patienten 3 vollständig in seiner Länge und Breite erfasst. Andere Ausgestaltungen des Sichtfeldes sind ebenfalls denkbar.

Der Computertomograph verfügt über einen Computer 12, welcher mit einer Anzeigeeinheit 11, beispielsweise zur graphischen Anzeige von Röntgenbildaufnahmen oder 2D-Bilddaten der 2D-Kamera, sowie einer Eingabeeinheit 7 verbunden ist. Bei der Anzeigeeinheit 11 kann es sich beispielsweise um einen LCD-, Plasma- oder OLED-Bildschirm handeln. Es kann sich weiterhin um einen berührungsempfindlichen Bildschirm handeln, welcher auch als Eingabeeinheit 7 ausgebildet ist. Ein solcher berührungsempfindlicher Bildschirm kann in das bildgebende Gerät integriert oder als Teil eines mobilen Geräts ausgebildet sein. Bei der Eingabeeinheit 7 handelt es sich beispielsweise um eine Tastatur, eine Maus, einen sogenannten "Touch-Screen" oder auch um ein Mikrofon zur Spracheingabe. Die Eingabeeinheit 7 kann auch eingerichtet sein, um Bewegungen eines Benutzers zu erkennen und in entsprechende Befehle zu übersetzen. Mittels Eingabeeinheit 7 kann beispielsweise ein Nutzer in den angezeigten 2D-Bilddaten die Position, Anordnung bzw. den Verlauf oder dergleichen eines virtuellen Szenebestandteils, hier beispielsweise in Form einer Grenzkonturlinie für einen Scanbereich, an die Röntgenbildaufnahmevorrichtung übergeben oder vorhandene Angaben dazu modifizieren.

Der Computer 12 steht mit der drehbaren Aufnahmeeinheit 17 zum Datenaustausch in Verbindung. Über die Verbindung 14 werden einerseits Steuersignale für die Röntgenbildaufnahme vom Computer 12 an die Aufnahmeeinheit 17 übertragen, andererseits können für den Patienten 3 aufgenommene Projektions-Daten für eine Bildrekonstruktion an den Computer 12 übertragen werden. Die Verbindung 14 ist in bekannter Weise kabelgebunden oder kabellos realisiert. Der Computer 12 ist ferner zum Austausch von Steuersignalen bzw. von Bilddaten auch mit der Kamera-Einheit 18 verbunden, insbesondere über dieselbe Verbindung 14.

Der Computer 12 weist eine Recheneinheit 16 auf. Die Recheneinheit 16 ist als Bild- bzw. Bilddatenbearbeitungseinheit ausgestaltet. Sie ist insbesondere eingerichtet, alle im Bezug zu dem erfindungsgemäßen Verfahren stehenden Rechenschritte an den per Kamera-Einheit 18 aufgenommenen Bilddaten durchzuführen. Die Recheneinheit 16 umfasst dazu eine Modell-Erstellungs-Einheit 21 zum Erstellen oder Erzeugen eines dreidimensionalen, virtuellen Szene-Modells aus den Tiefenbilddaten des Tiefenbildsensors 20, eine Einfüge-Einheit 22, die den z.B. durch den Nutzer eingegebenen, virtuellen Szene-bestandteil in dem Szene-Modell an der korrekten Position integriert und eine Erzeugungs-Einheit 23, die ein Ergebnisbild aus dem Szene-Modell umfassend den virtuellen Szene-Bestandteil erzeugt, indem sie dasselbe in die zweite Perspektive überführt. Dazu greift die Erzeugungs-Einheit 23 auf extrinsische und/oder intrinsische Kamera-Parameter zurück, die zum einen Lage-/Pose-Unterschiede der beiden Kameras und/oder jeweils intrinsische Abbildungseigenschaften oder Abbildungsfehler berücksichtigen. Die Kamera-Parameter können vorzugsweise in einem Speicher (nicht dargestellt) des Computers 12 oder dezentral abrufbar hinterlegt sein oder im Rahmen des erfindungsgemäßen Verfahrens ermittelt werden. Insbesondere Einfüge-Einheit 22 und Erzeugungs-Einheit 23 sind bevorzugt Teil einer bestehenden Rendering-Pipeline.

Die Recheneinheit 16 kann mit einem computerlesbaren Datenträger 13 zusammenwirken, insbesondere um durch ein Computerprogramm mit Programmcode ein erfindungsgemäßes Verfahren durchzuführen. Weiterhin kann das Computerprogramm auf dem maschinenlesbaren Träger abrufbar gespeichert sein. Insbesondere kann es sich bei dem maschinenlesbaren Träger um eine CD, DVD, Blu-Ray Disc, einen Memory-Stick oder eine Festplatte handeln. Die Recheneinheit 16, und damit auch ihre Sub-Komponenten, können in Form von Hard- oder in Form von Software ausgebildet sein. Beispielsweise ist die Recheneinheit 16 als ein sogenanntes FPGA (Akronym für das englischsprachige "Field Programmable Gate Array") ausgebildet oder umfasst eine arithmetische Logikeinheit.

In der hier gezeigten Ausführungsform ist auf dem Speicher des Computers 12 wenigstens ein Computerprogramm gespeichert, welches alle Verfahrensschritte des erfindungsgemäßen Verfahrens durchführt, wenn das Computerprogramm auf dem Computer 12 ausgeführt wird. Das Computerprogramm zur Ausführung der Verfahrensschritte des erfindungsgemäßen Verfahrens umfasst Programmcode. Weiterhin kann das Computerprogramm als ausführbare Datei ausgebildet sein und/oder auf einem anderen Rechensystem als dem Computer 12 gespeichert sein. Beispielsweise kann Röntgenbildaufnahmevorrichtung so ausgelegt sein, dass der Computer 12 das Computerprogramm zum Ausführen des erfindungsgemäßen Verfahrens über ein Intranet oder über das Internet in seinen internen Arbeitsspeicher lädt.

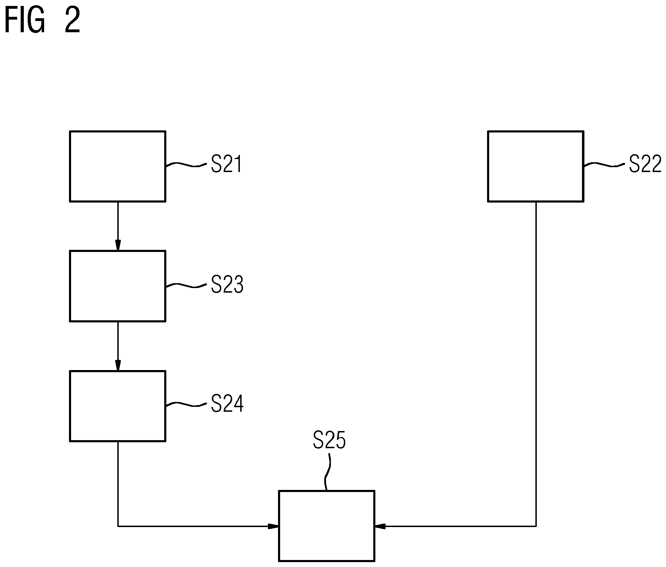

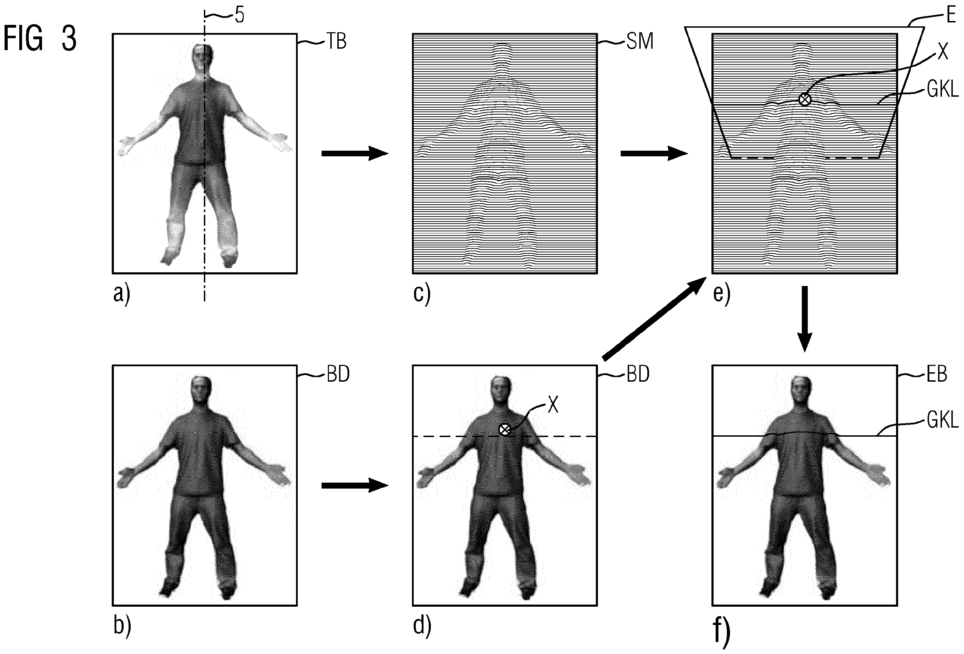

**Figur 2** beschreibt ein Ausführungsbeispiel des erfindungsgemäßen Verfahrens. In einem ersten Schritt S21 werden Tiefenbilddaten TB einer Realszene aus einer ersten Perspektive erfasst. Das Erfassen umfasst dabei einerseits das Aufnehmen der Tiefenbilddaten TB umfassend Tiefeninformation mittels eines Tiefenbildsensors 20 als auch bzw. nur das Übertragen von Tiefenbilddaten TB an eine weiterverarbeitende Recheneinheit 16. Die mittels Tiefenbilddaten TB erfasste Realszene bildet einen Patienten 3 ab, der auf dem Rücken auf einer Patientenliege 6 liegt. Die Tiefenbilddaten TB zeigen den Patienten 3 vollständig, können aber auch Bildinformation bezüglich der Patientenliege 6 und ggf. weiterer Szene-Bestandteile umfassen, die sich im Umfeld des abgebildeten Patienten und im Sichtfeld des Tiefenbildsensors befinden können, wie in **Figur 3a** schematisch und insbesondere nicht maßstabsgetreu dargestellt. Alternativ erfasst das Sichtfeld des Tiefenbildsensors lediglich einen Teil oder Bereich des Körpers des Patienten 3. Die Tiefenbilddaten TB umfassen zu jedem Bildpunkt bzw. zu jedem Pixel Tiefeninformation hinsichtlich der dreidimensionalen Oberflächenstruktur der Realszene, die einen Wert für den Abstand eines Objektpunktes zu dem Tiefenbildsensor 20 beschreibt. In den Tiefenbilddaten TB ist die Höhe mit einer Änderung von Graustufenwerten verknüpft. Die Graustufenwerte sind dort hoch (dunkel), wo z.B. der Patient 3 eine große Höhe aufweist, z.B. wie dargestellt im Brustbereich, im Bauchbereich, an den Armen oder auch im Gesicht. Die Graustufenwerte sind dort niedrig (hell), wo der Patient 3 eine geringe Höhe aufweist, z.B. im Halsbereich oder an den Beinen auf Kniehöhe. Dadurch ist insbesondere die Oberfläche des Patienten 3 in ihrer Form bzw. ihr Verlauf in der ersten Perspektive mit erfasst. Die Tiefenbilddaten TD werden im infraroten Wellenlängenbereich (780-1000 nm) aufgenommen.

In Schritt S22, der zeitgleich oder zumindest zeitnah zu Schritt S21 stattfindet, werden 2D-Bilddaten BD (**Figur 3b**) in Form eines Photos oder Videobildes erfasst. Dieses Erfassen umfasst einerseits das Akquirieren der 2D-Bilddaten BD mittels einer 2D- oder 3D-Kamera in einer zweiten Perspektive und andererseits oder nur das Übertragen derselben an die weiterverarbeitende Recheneinheit 16. Durch die zeitgleiche oder zeitlich nah bei einander liegende Aufnahme von Tiefenbilddaten TB und 2D-Bilddaten BD können Abweichungen von Bildinformation zwischen den Datensätzen, z.B. durch Bewegung des Patienten 3, vermieden bzw. verringert werden. Die 2D-Bilddaten BD werden im optischen Wellenlängenbereich (380-780 nm) erfasst.

Vorliegend sind erste und zweite Perspektive identisch, da Tiefenbildsensor 20 und 2D-Kamera 19 durch eine Kamera-Einheit 18 realisiert werden. Es besteht folglich Identität der extrinsischen Kamera-Parameter beider Kameras 19, 20. Jedoch sind für beide Kameras beliebige und insbesondere voneinander abweichende Posen und damit Aufnahmeperspektiven denkbar, die über die extrinsischen Kamera-Parameter exakt beschrieben und mittels geometrischen Transformationen, z.B. Translation oder Drehung, ineinander überführbar sind.

Die Modell-Erstellungseinheit 21 extrahiert in einem weiteren Schritt S23 die dreidimensionale Oberflächenform der Realszene umfassend den Patienten 3 aus den Tiefenbilddaten TB, indem sie die Tiefeninformation in ein virtuelles, dreidimensionales Oberflächenmodell SM überführt. Dazu wird beispielsweise eine den Oberflächenverlauf repräsentierende Punktwolke zu einer glatten dreidimensionalen Oberfläche interpoliert, die dann modellhaft, beispielsweise mittels aneinander gereihter Dreiecke, beschrieben wird. Alternativ beschreibt das Flächenmodell SM die durch die Oberfläche der Realszene gebildete Flächenkontur schichtweise entlang der Körperachse des Patienten 3, wie in **Figur 3c** exemplarisch veranschaulicht.

Es sei darauf hingewiesen, dass das Oberflächenmodell SM im Hinblick auf z.B. wegen einer Überdeckung fehlende, im den Tiefenbilddaten TB nicht enthaltene bzw. nicht dargestellte Bereiche der Szene-Oberfläche ergänzt werden kann, um ein vollständiges, mit anderen Worten geschlossenes, dreidimensionales Oberflächenmodell zu erhalten. Dies kann mittels bekannter Extrapolationsverfahren oder dergleichen erfolgen.

Dieses Vorgehen ermöglicht dann insbesondere auch Betrachtungen der Realszene aus anderen als der Aufnahmeperspektive der Kamera-Einheit 18, ohne dass sich dabei für den Betrachter störende Bildeindrücke aufgrund fehlender Bildinformation ergeben müssen. Hierzu sind jedoch ggf. Extrapolationen von 2D-Bildinformationen bezüglich der Oberfläche der Realszene erforderlich, um dem Benutzer einen sinnvollen Bildeindruck vermitteln zu können.

Alternativ und/oder zusätzlich kann die Modell-Erstellungseinheit 21 auch ein Volumen-Modell erstellen, wozu weitere Bilddaten aus anderen Datenquellen herangezogen werden können. Insbesondere können z.B. Röntgenbildaufnahmen des Patienten 3 aus früheren Untersuchungen zum Einsatz kommen, die beispielsweise innere Organe eines interessierenden Bereichs in ihrer Lage, Form, Größe und/oder physikalischen Eigenschaften oder dergleichen darstellen. Sofern erforderlich, kann ein räumlicher Bezug mittels bekannter Registrierungsverfahren zwischen Tiefenbilddaten und Röntgenbildaufnahmen hergestellt und dadurch eine korrekte Positionierung der zusätzlichen Bildinformation innerhalb des Szene-Modells erreicht werden.

Im vorliegenden Ausführungsbeispiel wird jedoch auf derartige Erweiterungen des Szene-Modells verzichtet.

In einem Schritt S24 wird wenigstens ein virtueller Szenebestandteil VSB in das dreidimensionale Szene-Modell SM eingefügt. Dazu muss zunächst eine geometrische Beschreibung des virtuellen Szene-Bestandteils VSB vorliegen bzw. erstellt werden. In diesem Ausführungsbeispiel soll eine Scanbereichsgrenze eingefügt werden, insbesondere eine Start-Linie für einen Scanbereich. Die Start-Linie liegt dabei in einer Start-Ebene, die in diesem Ausführungsbeispiel vertikal und senkrecht zur Patientenlängsachse 5 verläuft. In den 2D-Bilddaten BD würde eine Start-Ebene typsicherweise als gerade Linie (Strich-Linie in **Figur 3d**) dargestellt. Diese dient lediglich der beispielhaften Veranschaulichung und muss keiner tatsächlichen Start-Ebene entsprechen. Der Verlauf dieser Linie berücksichtigt jedoch nicht die perspektivischen Verzerrungen bzw. Abbildungsfehler der 2D-Bilddaten BD, sodass der Verlauf der geraden Linie in aller Regel nicht den exakten Verlauf der Schnittkurve zwischen Start-Ebene und Oberfläche der abgebildeten Realszene darstellt. Um diese Ungenauigkeiten zu beheben, kann vorgesehen sein, dem Nutzer die für bei der Untersuchungsplanung üblichen 2D-Bilddaten BD über die Anzeige-Einheit 11 zu präsentieren und er eine Position, auf der Patientenoberfläche, die innerhalb der Start-Ebene liegen soll, mittels Eingabeeinheit 7, insbesondere mittels Touch-Screen markiert. Dazu kann vorgesehen sein, dass er sich an anatomischen Landmarken oder aber zusätzlichen Referenz-Markierungen in der Realszene orientiert, die ihm bei der Markierung der Position helfen. Alternativ kann vorgesehen sein, dass der Computer 12 in Abhängigkeit der gewünschten Untersuchung und/oder des interessierenden Körperbereichs einen Vorschlag für eine Position macht, die der Nutzer per Eingabe bestätigen oder verschieben kann. Vorliegend wird beispielhaft eine Position X im Brustbereich des Patienten 3 markiert (**Figur 3d**), die innerhalb der Start-Ebene liegen soll. Nun kann ebenfalls in Schritt S24 die vertikal und senkrecht zur Patientenlängsachse 5 verlaufende Ebene E (**Figur 3e**) ermittelt werden, die die markierte Position X umfasst. Dazu können, im den allgemeinen Anwendungsfall extrinsische und/oder intrinsische Kamera-Parameter zum Einsatz kommen, um den korrekten, räumlichen Bezug zwischen 2D-Bilddaten BD und Tiefenbilddaten TB bzw. Szene-Modell SM herzustellen. Die durch die Position X definierte Ebene schneidet die Oberfläche des Patienten 3 bzw. der Realszene entsprechend Szene-Modell SM entlang einer gekrümmten Kurve, einer sogenannten Grenzkonturlinie GKL. Der Verlauf dieser Grenzkonturlinie GKL wird anhand des Szene-Modells SM ermittelt und im Szene-Modell SM eingefügt (**Figur 3e**).

In Schritt S25 wird durch die Erzeugungseinheit 23 ein Ergebnisbild EB (**Figur 3f**) aus dem Szene-Modell SM umfassend den virtuellen Szene-Bestandteil VSB für die Perspektive der 2D-Kamera 19 ermittelt, indem die 2D-Bilddaten BD entlang der Sichtstrahlen der 2D-Kamera 19 auf das Szene-Modell SM projiziert werden. Dadurch wird eine realistische, für den Benutzer gewohnte und insbesondere perspektivisch korrekte Darstellung der Grenzkonturlinie GKL erreicht. Hierbei können intrinsische Kamera-Parameter zum Einsatz kommen, um Abbildungsfehler der 2D-Kamera 19 und/oder des Tiefenbildsensors 20 zu kompensieren.

Das Ergebnisbild EB lässt sich bezüglich der perspektivisch dargestellten Bildinformation nicht von den 2D-Bilddaten BD gemäß Figur 3b unterscheiden und entspricht folglich einer Realszene-äquivalenten Darstellung. Jedoch verfügt das Ergebnisbild EB im Gegensatz zu den 2D-Bilddaten auch eine perspektivisch korrekte Darstellung der Grenzkonturlinie GKL. Diese ist dadurch gekennzeichnet, dass sich ihr Verlauf zumindest im Bereich des Patientenkörpers vom Verlauf der herkömmlichen Markierungslinie (Strich-Linie) für die Start-Ebene unterscheidet. Genau gesagt, ist sie gegenüber der Strich-Linie in Richtung Kopf des Patienten 3 entsprechend der Höheninformation aus den Tiefenbilddaten TB verschoben. Im Bereich der Liege 6 und des Außenumrisses des Patienten 3, fallen die Strich-Linie und die Grenzkonturlinie GKL jeweils aufeinander, weil die Höhen dort Null sind. Dabei wird der Einfachheit halber angenommen, dass die Höhe der Liege 6 als Basis "Null" in Höhenrichtung definiert ist. Deswegen ist die Liege 6 in den Tiefenbilddaten TB auch gleichförmig weiß, da alle Bereiche der Liege 6 in etwa auf gleichem Höhenniveau liegen.

Entsprechend gehören nun Bereiche des Patientenkörpers zum Scan-Bereich dazu, die zuvor fälschlicher Weise ausgeschlossen waren. Die Strich-Linie ist in Figur 3f lediglich zur besseren Anschaulichkeit der Unterschiede zur Grenzkonturlinie GKL mit abgebildet, im Ergebnisbild selbst jedoch nicht enthalten.

Selbstredend können beliebige andere Kamera-Positionen, insbesondere für 2D-Kamera und Tiefenbildsensor voneinander abweichende Positionen eingenommen werden, aus denen die Realszene umfassend den virtuellen Szene-Bestandteil VSB perspektivisch korrekt betrachtet werden können.

Ferner können weitere und/oder andere virtuelle Szenebestandteile als eine Scanbereichsgrenze für einen Scanbereich entsprechend der beschriebenen Vorgehensweise eingefügt werden.

Es wird darauf hingewiesen, dass es sich bei den vorhergehend detailliert beschriebenen Verfahren sowie bei der dargestellten Vorrichtung lediglich um Ausführungsbeispiele handelt, welche vom Fachmann in verschiedenster Weise modifiziert werden können, ohne den Bereich der Erfindung zu verlassen. Insbesondere können auch einzelne Schritte des erfindungsgemäßen Verfahrens eines Ausführungsbeispiels herausgegriffen und mit Verfahrensschritten anderer Ausführungsbeispiele kombiniert werden, sofern technisch möglich und sinnvoll. Obwohl beispielsweise die Erfindung für eine Nutzung an einer Computertomographie-Anlage beschrieben wurde, schließt dies nicht die vorteilhafte Nutzung an anderen medizintechnischen bildgebenden Anlagen nicht aus, wie beispielsweise
- anderen röntgenbasierten Anlagen, z. B. zur Erstellung von herkömmliche Röntgenaufnahmen oder Durchleuchtungen;
- Magnetresonanztomographie-Geräten (MRT);
- Szintigraphen, Positronen-Emissions-Tomographen (PET), Single-Photon-Emissionscomputertomographen (SPECT);
- Sonographen oder Farbdoppler-Geräten;
- diagnostische Thermographen;
- Elektrische Impedanz-Tomographen (EIT); oder
- Endoskopen.

Im Folgenden wird die vorliegende Erfindung nochmals kurz zusammengefasst. Erfindungsgemäß erfolgt eine virtuelle Modellierung aller Szene-Bestandteile, reell und virtuell. Ferner nutzt die Erfindung zur flexiblen Darstellung von virtuellen Szene-Bestandteilen bekannte und insbesondere eigenentwickelte Rendering-Pipelines aus. Dazu wird zunächst eine Realszene, in die ein virtueller Szene-Bestandteil integriert werden soll, aus zuvor erfassten 3D-Tiefeninformationen zu der Realszene, in ein dreidimensionales Modell überführt, also virtualisiert. Neben der Erfassung von 3D-Tiefeninformationen werden auch 2D-Bilddaten der Realszene erfasst, entweder aus der gleichen oder einer beliebigen anderen Perspektive. Unter Verwendung extrinsischer und/oder intrinsischer Kamera-Parameter aus der Kalibrierung der 2D-Kamera zur der 3D-Kamera werden die 2D-Bilddaten entlang der Sichtstrahlen auf das virtuelle, dreidimensionale Modell des Realszene projiziert. Wendet man die gleichen Kamera-Parameter auf eine sogenannte virtuelle "Renderer-Kamera" an, wird die virtualisierte Realszene unverfälscht, das heißt perspektivisch korrekt dargestellt und kann mit virtuellen Szene-Bestandteilen im Sinne einer Augmented Reality' angereichert werden. Ohne zusätzliche, virtuelle Szene-Bestandteile werden die Pixel eines Ergebnisbildes entsprechend den 2D-Bilddaten aus der Perspektive der 2D-Kamera exakt reproduziert. Die Freiheit, dieser Darstellung beliebige virtuelle Szene-Bestandteile hinzuzufügen, ermöglicht auch die Abbildung von komplexen Effekten wie die Beleuchtung durch einen Laser-Marker. Bei der Virtualisierung der Realszene kann z.B. für jeden Pixel eines RGBD-Bildes (Tiefenbilddaten) ein auf den Brennpunkt einer 2D-Kamera gerichtetes und entsprechend skaliertes Rechteck als virtueller Szenenbestandteil im aus den Tiefenbilddaten resultierenden Abstand zur 2D-Kamera modelliert werden. Die Rechtecke können entsprechend dem lokalen Gradienten der Tiefeninformation geneigt sein, um im weiteren Verlauf den gerichteten Anteil der Reflektion richtig zu modellieren, oder zu einem geschlossen Oberflächennetz verbunden werden. Ein Renderer bzw. eine Graphik-Pipeline erstellt nun aus beliebiger Perspektive eine Ansicht der Szene. Wird die virtuelle Kamera unter Berücksichtigung bekannter Kameraparameter (z.B. Field-of-View, Bildverzerrung, etc.) in Deckung mit der 2D-Kamera gebracht, stellen sich die 2D-Bilddaten unverfälscht dar, da sich die Rechtecke aus der Originalperspektive nahtlos nebeneinander darstellen. Beliebige weitere, virtuelle Szenenbestandteile werden durch den Renderer korrekt, d.h. hinsichtlich Abschattungen, etc., relativ zu den reellen, modellierten Szenenbestandteilen dargestellt. Der somit erreichte Bildeindruck des Ergebnisbildes ist aufgrund der Berücksichtigung der perspektivischen Verzerrung der 2D-Kamera nicht von den 2D-Bilddaten zu unterscheiden.

## Patentansprüche

1. Verfahren zum perspektivischen Darstellen wenigstens eines virtuellen, innerhalb einer Realszene angeordneten Szenebestandteils (VSB) mittels eines Ergebnisbildes (EB), umfassend
- Erfassen (S21) von Tiefenbilddaten (TB) der Realszene aus einer ersten Perspektive mittels Tiefenbildsensor (20),
- Erfassen (S22) von 2D-Bilddaten (BD) der Realszene aus einer zweiten Perspektive mittels 2D-Kamera (19),
- Erstellen (S23) eines virtuellen, dreidimensionalen Szene-Modells (SM) der Realszene unter Berücksichtigung von Tiefeninformation der Tiefenbilddaten,
- Einfügen (S24) wenigstens eines virtuellen Szenebestandteils in das dreidimensionale, virtuelle Szene-Modell,
- Erzeugen eines Ergebnisbildes durch perspektivisches Projizieren der 2D-Bilddaten entsprechend der zweiten Perspektive auf das virtuelle, dreidimensionale Szene-Modell umfassend den virtuellen Szene-Bestandteil,
wobei die Realszene ein auf einer Patientenliege (6) einer medizinischen Bildgebungsanlage befindliches Untersuchungsobjekt (3) umfasst und der wenigstens eine virtuelle Szenebestandteil einer Scanbereichsgrenze SBG, welche in Form einer Grenzkonturlinie GKL ausgebildet ist, entspricht, wobei für den Scanbereich mit der medizinischen Bildgebungsanlage Bilddaten erfasst werden sollen und wobei die Scanbereichsgrenze SBG einer virtuell auf das Untersuchungsobjekt projizierten Licht- oder Laser-Markierung entspricht.

2. Verfahren nach Anspruch 1, wobei das perspektivische Projizieren mittels eines Renderers erfolgt.

3. Verfahren nach Anspruch 1 oder 2, wobei das perspektivische Projizieren unter Berücksichtigung extrinsischer und/oder intrinsischer Kamera-Parameter der 2D-Kamera und/oder des Tiefenbildsensors erfolgt.

4. Verfahren nach einem der vorstehenden Ansprüche, wobei die 2D-Bilddaten und die Tiefenbilddaten aus der gleichen Perspektive erfasst werden.

5. Verfahren nach einem der vorstehenden Ansprüche, wobei das virtuelle, dreidimensionale Szene-Modell ein Oberflächenmodell ist.

6. Verfahren nach einem der vorstehenden Ansprüche, wobei das Einfügen des virtuellen Szene-Bestandteils in das virtuelle, dreidimensionale Szene-Modell anhand von Benutzereingaben erfolgt.

7. Verfahren nach Anspruch 6, wobei die Anordnung des virtuellen Szenebestandteils unter Verwendung der 2D-Bilddaten ermittelt wird.

8. Verfahren nach einem der vorstehenden Ansprüche, wobei die 2D-Bilddaten der Realszene im optischen/sichtbaren Wellenlängenbereich erfasst werden.

9. Verfahren nach einem der vorstehenden Ansprüche, wobei die Tiefenbilddaten der Realszene im Infrarot-Wellenlängenbereich erfasst werden.

10. Vorrichtung zum perspektivischen Darstellen wenigstens eines virtuellen, innerhalb einer Realszene angeordneten Szenebestandteils (VSB) mittels eines Ergebnisbildes (EB), umfassend
- einen Tiefenbildsensor (20), welcher Tiefenbilddaten (TB) der Realszene aus einer ersten Perspektive erfasst,
- eine 2D-Kamera (19), welche 2D-Bilddaten (BD) der Realszene aus einer zweiten Perspektive erfasst,
- eine Modell-Erstellungseinheit (21), die ein virtuelles, dreidimensionales Szene-Modell (SM) der Realszene unter Berücksichtigung von Tiefeninformation der Tiefenbilddaten erstellt,
- eine Einfüge-Einheit (22), die wenigstens einen virtuellen Szenebestandteil in das dreidimensionale, virtuelle Szene-Modell einfügt,
- eine Erzeugungs-Einheit (23), die ein Ergebnisbild durch perspektivisches Projizieren der 2D-Bilddaten entsprechend der zweiten Perspektive auf das virtuelle, dreidimensionale Szene-Modell umfassend den virtuellen Szene-Bestandteil erzeugt
wobei die zu erfassende Realszene ein auf einer Patientenliege (6) einer medizinischen Bildgebungsanlage befindliches Untersuchungsobjekt (3) umfasst und der wenigstens eine virtuelle Szenebestandteil einer Scanbereichsgrenze SBG, welche in Form einer Grenzkonturlinie GKL ausgebildet ist, entspricht, wobei für den Scanbereich mit der medizinischen Bildgebungsanlage Bilddaten erfasst werden sollen und wobei die Scanbereichsgrenze SBG einer virtuell auf das Untersuchungsobjekt projizierten Licht- oder Laser-Markierung entspricht.

11. Vorrichtung nach Anspruch 10, wobei die 2D-Kamera und der Tiefenbilddatensensor zu einer Kamera-Einheit (18) zusammengefasst sind.

12. Medizinische Bildgebungsanlage, insbesondere Computertomographiegerät, umfassend die Vorrichtung nach einem der Ansprüche 10 oder 11.

## Claims

1. Method for the perspective representation, by means of an output image (OI), of at least one virtual scene component (VSC) which is arranged within a real scene, wherein said method comprises:
- capturing (S21) depth image data (DD) of the real scene from a first perspective by means of a depth image sensor (20);
- capturing (S22) 2D image data (ID) of the real scene from a second perspective by means of a 2D camera (19);
- creating (S23) a virtual three-dimensional scene model (SM) of the real scene with reference to depth information from the depth image data;
- inserting (S24) at least one virtual scene component into the three-dimensional virtual scene model; and
- generating an output image by means of perspective projection of the 2D image data corresponding to the second perspective onto the virtual three-dimensional scene model comprising the virtual scene component,
wherein the real scene comprises an examination object (3) which is situated on a patient couch (6) of a medical imaging facility, and the at least one virtual scene component corresponds to a scan region boundary SRB, which is designed in the form of a boundary contour line BCL, wherein image data is to be captured for the scan region by the medical imaging facility and wherein the scan region boundary SRB corresponds to a light marking or laser marking which is virtually projected onto the examination object.

2. Method according to claim 1, wherein the perspective projection is effected by means of a renderer.

3. Method according to claim 1 or 2, wherein the perspective projection is effected with reference to extrinsic and/or intrinsic camera parameters of the 2D camera and/or of the depth image sensor.

4. Method according to one of the preceding claims, wherein the 2D image data and the depth image data are captured from the same perspective.

5. Method according to one of the preceding claims, wherein the virtual three-dimensional scene model is a surface model.

6. Method according to one of the preceding claims, wherein the insertion of the virtual scene component into the virtual three-dimensional scene model is effected on the basis of user inputs.

7. Method according to claim 6, wherein the arrangement of the virtual scene component is determined using the 2D image data.

8. Method according to one of the preceding claims, wherein the 2D image data of the real scene is captured in the optical/visible wavelength range.

9. Method according to one of the preceding claims, wherein the depth image data of the real scene is captured in the infrared wavelength range.

10. Device for the perspective representation, by means of an output image (OI), of at least one virtual scene component (VSC) which is arranged within a real scene, wherein said device comprises:
- a depth image sensor (20) which captures depth image data (DD) of the real scene from a first perspective;
- a 2D camera (19) which captures 2D image data (ID) of the real scene from a second perspective;
- a model-creation unit (21) which creates a virtual three-dimensional scene model (SM) of the real scene with reference to depth information from the depth image data;
- an insertion unit (22) which inserts at least one virtual scene component into the three-dimensional virtual scene model; and
- a generation unit (23) which generates an output image by means of perspective projection of the 2D image data corresponding to the second perspective onto the virtual three-dimensional scene model comprising the virtual scene component
wherein the real scene to be captured comprises an examination object (3) which is situated on a patient couch (6) of a medical imaging facility, and the at least one virtual scene component corresponds to a scan region boundary SRB, which is designed in the form of a boundary contour line BCL, wherein image data is to be captured for the scan region by the medical imaging facility and wherein the scan region boundary SRB corresponds to a light marking or laser marking which is virtually projected onto the examination object.

11. Device according to claim 10, wherein the 2D camera and the depth image data sensor are combined to form a camera unit (18).

12. Medical imaging facility, in particular a computer tomography apparatus, comprising the device according to one of claims 10 or 11.

## Revendications

1. Procédé de représentation en perspective d'au moins une partie (VSB) virtuelle de constitution d'une scène, disposée dans une scène réelle, au moyen d'une image (EB) de résultat, comprenant
- la saisie (S21) de données (TB) d'image profonde de la scène réelle à partir d'une première perspective au moyen d'un capteur (20) d'image profonde,
- la saisie (S22) de données (BD) d'image en 2D de la scène réelle à partir d'une deuxième perspective au moyen d'une caméra (19) en 2D,
- l'établissement (S23) d'un modèle (SM) virtuel de scène en trois dimensions de la scène réelle, en prenant en compte l'information de profondeur des données d'image profonde,
- l'insertion (S24) d'au moins une partie virtuelle de constitution de la scène dans le modèle virtuel de scène en trois dimensions,
- la production d'une image de résultat, par une projection en perspective de la donnée d'image en 2D correspondant à la deuxième perspective, sur le modèle virtuel de scène en trois dimensions comprenant la partie virtuelle de constitution d'une scène, dans lequel la scène réelle comprend un objet (3) à examiner se trouvant sur une couchette (6) de patient d'une installation d'imagerie médicale et la au moins une partie virtuelle de constitution d'une scène correspond à une limite SBG d'une zone de balayage, qui est sous la forme d'une ligne GKL de contour limite, dans lequel, pour la zone de balayage, des données d'image doivent être saisies par l'installation d'imagerie médicale, et dans lequel la limite SBG de zone de balayage correspond à un marquage lumineux ou laser projeté virtuellement sur l'objet à examiner.

2. Procédé suivant la revendication 1, dans lequel la projection en perspective s'effectue au moyen d'un rendu.

3. Procédé suivant la revendication 1 ou 2, dans lequel la projection en perspective s'effectue en prenant en compte des paramètres extrinsèques et/ou intrinsèques de la caméra en 2D et/ou du capteur d'image profonde.

4. Procédé suivant l'une des revendications précédentes, dans lequel on saisit les données d'image en 2D et les données d'image profonde à partir de la même perspective.

5. Procédé suivant l'une des revendications précédentes, dans lequel le modèle virtuel de scène en trois dimensions est un modèle de surface.

6. Procédé suivant l'une des revendications précédentes, dans lequel l'insertion de la partie virtuelle de constitution de la scène dans le modèle virtuel de scène en trois dimensions s'effectue à l'aide d'entrées d'utilisateur.

7. Procédé suivant la revendication 6, dans lequel on détermine la position de la partie virtuelle de constitution de la scène en utilisant les données d'image en 2D.

8. Procédé suivant l'une des revendications précédentes, dans lequel on saisit les données d'image en 2D de la scène réelle dans le domaine optique / visible des longueurs d'onde.

9. Procédé suivant l'une des revendications précédentes, dans lequel on saisit les données d'image profonde de la scène réelle dans le domaine infrarouge des longueurs d'onde.

10. Dispositif de représentation en perspective d'au moins une partie (VSB) virtuelle de constitution d'une scène disposée dans une scène réelle, au moyen d'une image (EB) de résultat, comprenant
- un capteur (20) d'image profonde, qui saisit des données (TB) d'image profonde de la scène réelle à partir d'une première perspective,
- une caméra (19) en 2D, qui saisit des données (BD) d'image en 2D de la scène réelle à partir d'une deuxième perspective,
- une unité (21) d'établissement d'un modèle, qui établit un modèle (SM) de scène virtuel en trois dimensions de la scène réelle, en tenant compte de l'information de profondeur des données d'image profonde,
- une unité (22) d'insertion, qui insère au moins une partie virtuelle de constitution de scène dans le modèle de scène virtuel en trois dimensions,
- une unité (23) de production, qui produit une image de résultat par projection en perspective des données d'image en 2D correspondant à la deuxième perspective sur le modèle de scène virtuel en trois dimensions comprenant la partie virtuelle de constitution de scène, dans lequel la scène réelle à détecter comprend un objet (3) à examiner se trouvant sur une couchette (6) de patient d'une installation d'imagerie médicale et la au moins une partie virtuelle de constitution de scène correspond à une limite SBG de balayage, qui est constitué d'une ligne GKL de contour limite, dans lequel, pour la zone de balayage, des données d'image doivent être saisies par l'installation d'imagerie médicale et dans lequel la limite SBG de zone de balayage correspond à un marquage de lumière ou laser projeté virtuellement sur l'objet à examiner.

11. Dispositif suivant la revendication 10, dans lequel la caméra en 2D et le capteur de données d'image profonde sont rassemblés en une unité (18) de caméra.

12. Installation d'imagerie médicale, en particulier appareil de tomodensitométrie assisté par ordinateur comprenant le dispositif suivant l'une des revendications 10 ou 11.
